# EUROPEAN PATENT SPECIFICATION

(11) **EP 4 393 390 B1**
(45) Date of publication and mention of the grant of the patent: **09.04.2025**
(21) Application number: 23219875.4
(22) Date of filing: 22.12.2023
(51) Int. Cl.: A61B 5/06, A61B 34/20, A61B 5/00

(54) **TILT DETECTION FOR A BASKET CATHETER**
NEIGUNGSDETEKTION FÜR EINEN KORBKATHETER
DÉTECTION D'INCLINAISON POUR UN CATHÉTER À PANIER

(30) Priority: 29.12.2022 US 202218090785
(43) Date of publication of application: 03.07.2024
(73) Proprietor: BIOSENSE WEBSTER (ISRAEL) LTD., 2066717 Yokneam (IL)
(72) Inventor: GOVARI, Assaf, 2066717 Yokneam (IL); BEECKLER, Christopher Thomas, Irvine, 92618 (US); ALTMANN, Andres Claudio, 2066717 Yokneam (IL)
(74) Representative: Carpmaels & Ransford LLP

(56) References cited:
- EP-A1- 3 673 799
- US-A1- 2019 350 489
- US-A1- 2020 155 224
- US-A1- 2022 192 753

## Description

### FIELD OF THE INVENTION

The present invention relates to catheters generally and to basket and balloon catheters in particular.

### BACKGROUND OF THE INVENTION

US 2019/0350489 A1 discloses a method that includes, in a processor, receiving position signals that are indicative of positions of (i) multiple electrodes disposed on an inflatable balloon fitted at a distal end of a catheter, and (ii) first and second electrodes fitted on a shaft of the catheter, on either side of the balloon. The positions of the multiple electrodes disposed on the balloon are calculated based on the received position signals and based on a known distance between the first and second electrodes.

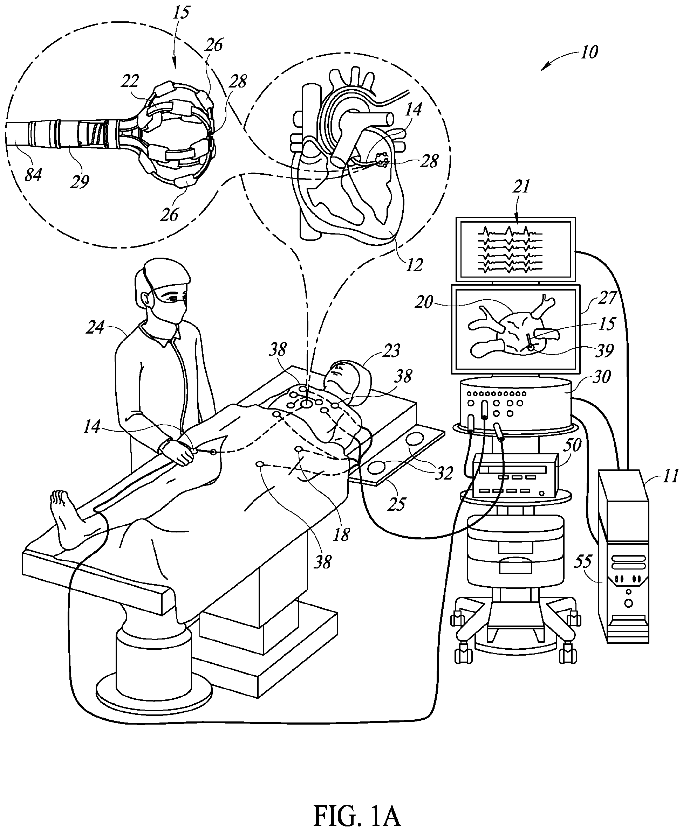

Ablation of body tissue, such as cardiac tissue is known. Reference is made to Fig. 1A showing an exemplary catheter-based electro-anatomical (EA) mapping and ablation system 10. System 10 includes multiple catheters, which are percutaneously inserted by a physician 24 through the patient's vascular system into a chamber or vascular structure of a heart 12. Typically, a delivery sheath catheter is inserted into the left or right atrium near a desired location in heart 12. Thereafter, one or more catheters may be inserted into delivery sheath catheter(s) so as to arrive at the desired location in heart 12. The plurality of catheters may include catheters dedicated for sensing Intracardiac Electrogram (IEGM) signals, catheters dedicated for ablating and/or catheters dedicated for both sensing and ablating. An exemplary catheter 14 that is configured for ablating is illustrated herein. Physician 24 may place a distal end of an ablation catheter in contact with a target site for ablating tissue.

Catheter 14 is an exemplary basket catheter that includes a basket distal assembly 15 having one and preferably multiple electrodes 26 optionally distributed over a plurality of splines 22. Other ablating catheters include balloon catheters having balloon distal assemblies. Basket catheter 14 may additionally include a position sensor 29 embedded in a shaft 84 to which basket distal assembly 15 is attached, for tracking position and orientation of a distal tip 28 of basket distal assembly 15. Optionally and preferably, position sensor 29 is a magnetic based position sensor including three magnetic coils for sensing three-dimensional (3D) position and orientation.

Magnetic based position sensor 29 may be operated together with a location pad 25 including a plurality of magnetic coils 32 configured to generate magnetic fields in a predefined working volume. Real time position of distal tip 28 of catheter 14 may be tracked based on magnetic fields generated with location pad 25 and sensed by magnetic based position sensor 29. Details of the magnetic based position sensing technology are described in U.S. Patent Nos. 5,5391,199; 5,443,489; 5,558,091; 6,172,499; 6,239,724; 6,332,089; 6,484,118; 6,618,612; 6,690,963; 6,788,967; 6,892,091.

System 10 includes one or more electrode patches 38 positioned for skin contact on patient 23 to establish location reference for location pad 25 as well as impedance-based tracking of electrodes 26. Impedance based tracking of electrodes 26 is also referred to as Active Current Location (ACL) tracking. The ACL method is implemented in various medical applications, for example, in the CARTO^{™} system, produced by Biosense-Webster Inc. (Irvine, California).

For impedance-based tracking, electrical current is directed to electrodes 26 and sensed at electrode skin patches 38 so that the location of each electrode can be triangulated via the electrode patches 38. Details of the impedance-based location tracking technology (ACL tracking) are described in US Patent Nos. 7,536,218; 7,756,576; 7,848,787; 7,869,865; and 8,456,182.

System 10 may include an ablation energy generator 50 that is adapted to conduct ablative energy to one or more of electrodes 26. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 30 is an interface configured to establish electrical communication between catheters, other electrophysiological equipment, power supply and a workstation 55 for controlling operation of system 10. Optionally and preferably, PIU 30 additionally includes processing capability for implementing real-time computations of location of the catheters and for performing ECG calculations.

Workstation 55 includes a memory, a processor unit with memory or storage with appropriate operating software stored therein, and a user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of multiple catheters within the heart chamber (Fig. 1A shows an icon of catheter 14 and its recent ablation area 39 positioned in relation to EA map 20), and (4) displaying on display device 27 sites of interest such as places where ablation energy has been applied. One commercial product embodying elements of system 10 is available as the CARTO^{™} 3 System, available from Biosense Webster, Inc., 31 Technology Drive, Suite 200, Irvine, CA 92618 USA.

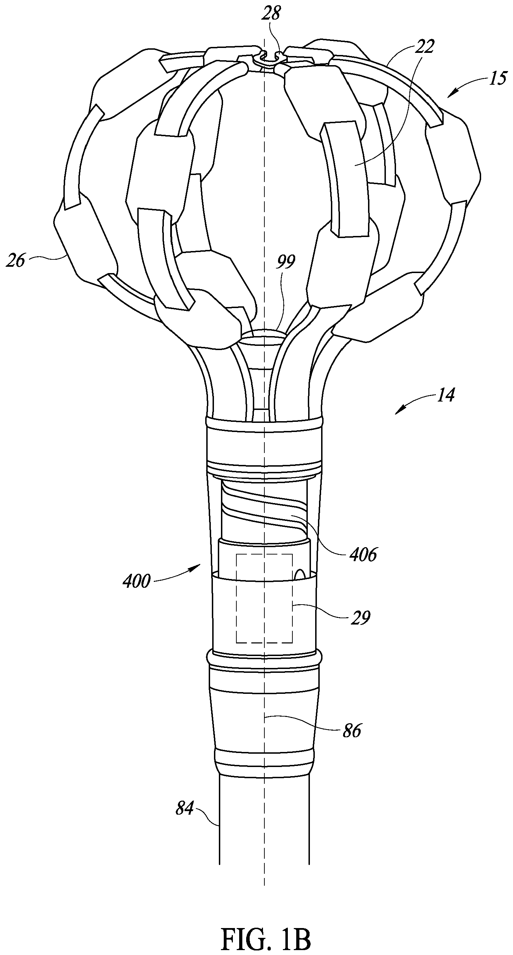

Reference is made to Fig. 1B, which illustrates an exemplary basket catheter 14 in an expanded form in which splines 22 bow radially outwardly. In a collapsed form, not shown, splines 22 are arranged generally along a longitudinal axis 86 of tubular shaft 84. Basket distal assembly 15 expands to a spherical or near spherical shape with a diameter of 15 mm or more. Basket catheter 14 is typically deflectable at a vicinity of an interface between shaft 84 and distal assembly 15 where, in Fig. 1B, there is a joint connection. In the embodiment of Fig. 1B, basket catheter 14 includes a contact force sensor assembly 400 at the vicinity of the interface to determine contact force of splines 22 against cardiac tissues. The force sensing capability may be added to ablation catheters to provide indication that sufficient contact with the tissue has been established before delivering the ablation energy.

Contact force sensor assembly 400 includes a helical spring 406 integrated as part of shaft 84 and/or disposed inside shaft 84. Typically, helical spring 406 is positioned as close as possible to a distal assembly, which may be basket distal assembly 15 or a balloon assembly. Contact force applied on cardiac tissue by the electrodes 26 actuate compression and/or bending of helical spring 406. Compression and/or bending is sensed by a transmitting circuit and sensing circuit located on opposite ends of helical spring 406 (e.g., opposite ends along longitudinal axis 86) and related to contact force. The transmitting circuit and sensing circuit are not shown here for simplicity purposes. An example contact-force sensor assembly 400 is described for example in U.S. Patent No. 8,333,103 entitled "Calibration of a force measuring system for large bend angles of a catheter" and/or U.S. Patent No. 9,050,105 entitled "Catheter with multiple irrigated electrodes and a force sensor." Typically, helical spring 406 may bend up to 30 degrees in one or more directions depending on the force applied or optionally up to 45 degrees in one or more directions.

### Summary of the Invention

In accordance with the present invention there is provided a method implemented by a processor of an electro-anatomical mapping system as claimed in claim 1 and an electrode location determiner for an electro-anatomical mapping system as claimed in claim 8. Embodiments of the present invention are provided in the dependent claims.

### BRIEF DESCRIPTION OF THE DRAWINGS

The subject matter regarded as the invention is particularly pointed out and distinctly claimed in the concluding portion of the specification. The invention, however, both as to organization and method of operation, together with objects, features, and advantages thereof, may best be understood by reference to the following detailed description when read with the accompanying drawings in which:
Fig. 1A is a schematic illustration of an exemplary catheter-based electro-anatomical (EA) mapping and ablation system;
Fig. 1B is a schematic illustration of an exemplary basket catheter in an expanded form, useful in the system of Fig. 1A;
Fig. 2 is a schematic illustration of the basket catheter of Fig. 1B bent against a tissue surface to be mapped or ablated;
Fig. 3 is a block diagram illustration of an electrode location determiner forming part of the system of Fig. 1A;
Fig. 4 is a flow chart illustration of a method implemented by the electrode location determiner of Fig. 3; and
Figs. 5A and 5B are schematic illustrations of the basket catheter of Fig. 1B in straight and bent positions, respectively.

It will be appreciated that for simplicity and clarity of illustration, elements shown in the figures have not necessarily been drawn to scale. For example, the dimensions of some of the elements may be exaggerated relative to other elements for clarity. Further, where considered appropriate, reference numerals may be repeated among the figures to indicate corresponding or analogous elements.

### DETAILED DESCRIPTION OF THE PRESENT INVENTION

In the following detailed description, numerous specific details are set forth in order to provide a thorough understanding of the invention. However, it will be understood by those skilled in the art that the present invention may be practiced without these specific details. In other instances, well-known methods, procedures, and components have not been described in detail so as not to obscure the present invention.

Applicant has realized that for catheters with large distal assemblies, such as basket assemblies and balloon assemblies, bending at a distal end of the shaft due to pressing as the catheter against the body tissue may lead to significant deflection of the distal assembly with respect to the shaft.

Applicant has further realized that the significant deflection is at least partially due to the relatively large size of the distal end assembly. Due to its size, the distal assembly may have a moment arm of 15 mm or more, e.g., 15 mm - 30 mm. The bend angle of the distal assembly may be especially significant for catheters that include a helical spring on its shaft.

In known systems, when displaying visual indicia of a catheter on an EA map displayed on display 27 (Fig. 1A) in real time, the visual indicia is positioned at the location determined based on the output of the position sensor mounted on the shaft proximal to the bending point. This location is selected since it includes a magnetic based position sensor that can track location with high accuracy, e.g., +/- 1 mm accuracy. However, Applicant has realized that for relatively large distal assemblies mounted on a shaft that accommodates bending, such as for basket catheter 14 of Fig. 1B, there may be a significant discrepancy between a location of position sensor on the shaft and a distal end of distal assembly and/or locations of electrodes on the distal assembly.

Applicant has realized that ACL tracking of the electrodes on the distal assembly may be used for improving the ability to track the location of the distal assembly when bending is expected. Although ACL tracking is known to have limited accuracy, Applicant has realized that this may be due to its use of individual ACL determined locations of each of a plurality of electrodes on the distal assembly. Applicant has further realized that since basket and balloon catheters may be assumed to have a rigid assembly, combining the individual ACL determined locations to compute a center of mass of the distal assembly may provide significantly better accuracy for the center of mass location, which may then be utilized to infer the location of each of the electrodes on the distal assembly.

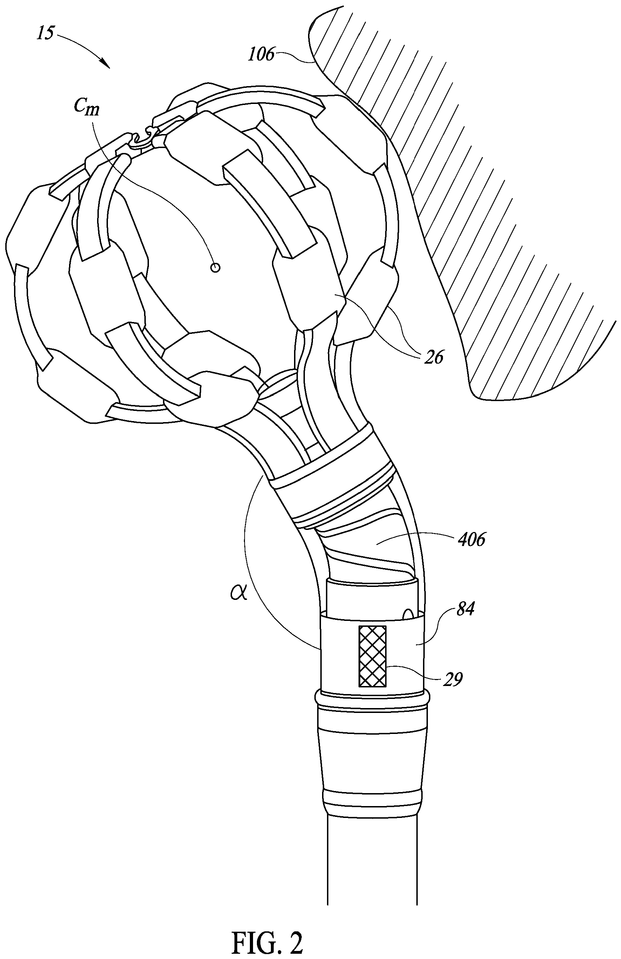

An example deflection is shown in Fig. 2, to which reference is now made. Fig. 2 shows basket catheter 14 of Fig. 1B, along with its multiple electrodes 26, shaft 84 and helical spring 406 and magnetic sensor 29, known as a triaxial sensor (TAS). Magnetic sensor 29 is longitudinally mounted on shaft 84, next to contact force assembly 400. Fig. 2 shows basket distal assembly 15 against a tissue surface 106 to be ablated and the resulting bend in spring 406 with a tilt angle α.

Applicant has further realized that, even though basket catheter 14 may be an ablation catheter, it has the capability to provide the ACL locations of its electrodes 26, using an active current location (ACL) method of determining electrode location.

As mentioned hereinabove, while the 3D location and orientation of shaft 84 may be determined with relatively high accuracy, e.g., +/- 1 mm accuracy, 3D location of each of electrodes 26 based on the ACL tracking is significantly less accurate. As a result, ACL tracking may not be utilized to visually indicate on display 27 the location of basket distal assembly 15 or other distal assembly on the rendered EA map 20 (Fig. 1A) and/or visually indicating on display 27 places where ablation energy has been applied by one or more electrodes 26. The locations cannot be easily inferred from the output of position sensor 29 due to the ability of basket catheter 14 or other type of catheter to bend in different directions with respect to the orientation of shaft 84 on which position sensor 29 is mounted.

Applicant has realized that the ACL locations may be sufficiently accurate when averaging the ACL locations of multiple electrodes 26, and has further realized that such an average may provide a simple and sufficiently accurate method for determining a location of a center Cₘ of mass of basket distal assembly 15. Based on the determined center of mass and assuming that basket distal assembly 15 or other distal assembly maintains its shape during contact with the tissue, 3D position of each of the electrodes on basket distal assembly 15 or other distal assembly may be computed.

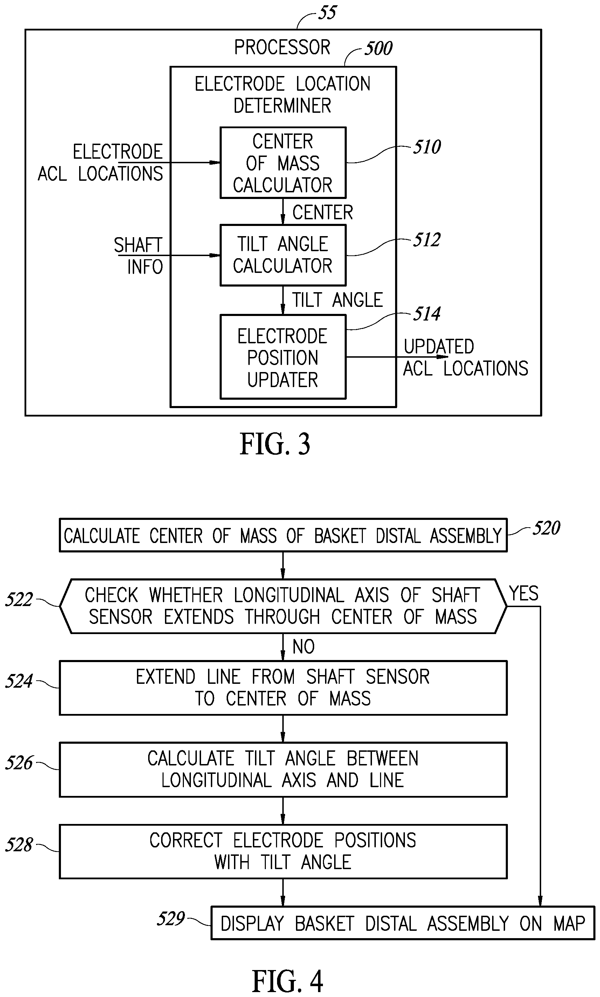

In accordance with a preferred embodiment of the present invention and in reference to Fig. 3, processor 55 comprises an electrode location determiner 500 which utilizes the center Cₘ of mass of basket distal assembly 15 and known geometry of basket distal assembly 15 to track the 3D location of each of electrodes 26, e.g., to update or correct the three-dimensional ACL locations (xᵢ,yᵢ,zᵢ) of electrodes 26. Electrode location determiner 500 comprises a center of mass calculator 510, a tilt angle calculator 512 and an electrode position updater 514. Reference is also made to Fig. 4, which illustrates a method implemented by electrode location determiner 500.

Center of mass calculator 510 receives the ACL locations (xᵢ,yᵢ,zᵢ), defined in a coordinate system of location pad 25. Center of mass calculator 510 computes (step 520 of Fig. 4) the location of center Cₘ of mass of basket distal assembly 15 therefrom. For example, since electrodes 26 are generally spread symmetrically around center Cₘ, and since basket distal assembly 15 may be considered to maintain its shape when pressed against tissue 106, the location of center Cₘ may be determined by the average of the ACL locations of electrodes 26. Alternatively, for a basket assembly including an electrode arrangement that is not symmetrical, location of center Cm may be determined based on the known positioning of the electrodes on that basket assembly.

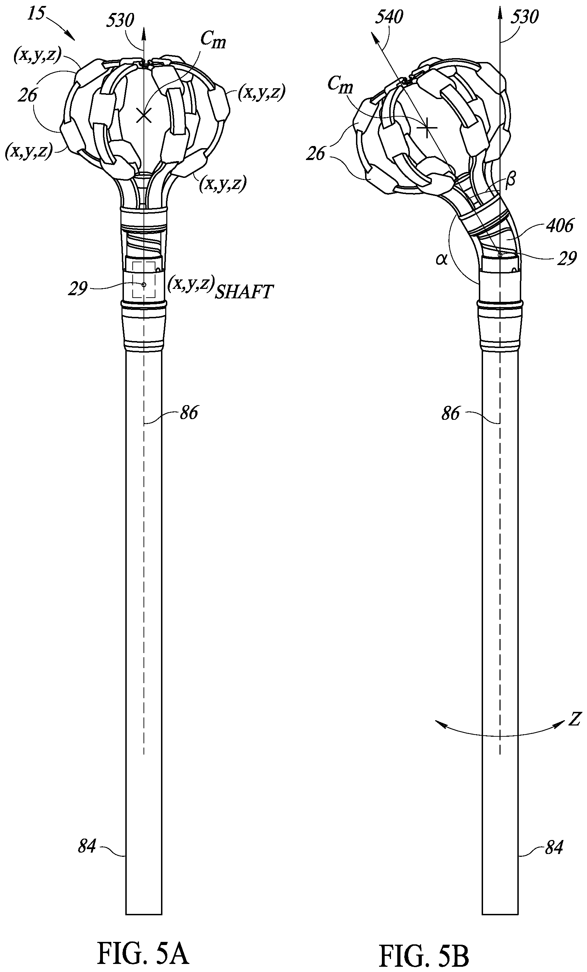

Reference is now made to Figs. 5A and 5B which illustrate the operation of tilt angle calculator 512. Tilt angle calculator 512 utilizes center Cₘ of mass together with the output of position sensor 29 to determine tilt angle α. Position sensor 29 generates a position of shaft 84 in six degrees of freedom, e.g., three-dimensional position (x,y,z) as well as its three-dimensional rotation (ϕ, θ, Ψ) in the coordinate system defined by location pad 25.

Fig. 5A shows shaft 84 with distal assembly 15 fixed at its distal end thereof, aligned with shaft 84. As a result, a line 530 drawn through the location of position sensor 29, along a longitudinal axis 86, extends to center Cₘ of mass of basket distal assembly 15. Thus, tilt angle calculator 512 initially checks (in step 522 of Fig. 4) whether longitudinal axis 86 extends through center Cₘ of mass as computed by center of mass calculator 510. If there is little error, then tilt angle α is minimal or 0 and electrode location determiner 500 may display (step 529) basket distal assembly 15 at its location on map 20 (Fig. 1A).

Otherwise, the situation of Fig. 5B applies, in which center Cₘ of mass is deflected from longitudinal axis 86. To determine tilt angle α, tilt angle calculator 512 extends (step 524) a line 540 from the location (x,y,z)_{SHAFT} of position sensor 29, here acting as a shaft sensor, to the computed center Cₘ of mass and then determines (step 526) the angle therebetween in the 3D coordinate system of location pad 25. Tilt angle calculator 512 may implement vector angle calculations, as is known in the art, to determine the angle. As can be seen from Fig. 5B, that angle is angle β, which is the supplementary angle of tilt angle α defining the deflection of basket catheter 14.

Electrode position updater 514 may then combine tilt angle α with the three-dimensional position (x,y,z)_{SHAFT} of sensor 29 as well as its three-dimensional rotation (ϕ, θ, Ψ) around its longitudinal axis 86 to determine (step 528) the locations (xᵢ,yᵢ,zᵢ) of electrodes 26. Electrode position updater 514 may assume that splines 22 will not bend significantly when pressed against tissue 106, giving basket catheter 14 a rigid construction. Optionally, electrode position updater 514 may first update the ACL location of distal end 28. Since the other ACL locations are defined with respect to distal end 28, updater 514 may determine the ACL locations and/or may update the ACL locations of electrodes 26 from the computed location of distal end 28.

Electrode position updater 514 may then display (step 529) basket distal assembly 15 on the anatomical map 20 displayed on display device 27 with improved accuracy. During a mapping procedure, this may lead to a more accurate map. Furthermore, during an ablation procedure when one or more of electrodes 26 delivers ablation energy, the electrode position updater 514 may more accurately display the locations at which an ablation event took place. It will be appreciated that, although each ACL location (xᵢ, yᵢ, zᵢ) is not particularly accurate, the present invention, using the accumulated output from all or most of electrodes 26, may provide a sufficiently accurate estimation of the location and angular deflection of center Cₘ of mass with respect to the location and angular positioning of position sensor 29.

Moreover, it will be appreciated that electrode location determiner 500 may operate on each set of ACL electrode locations which may be received. Since these ACL electrode locations may be received in real-time, electrode location determiner 500 may operate in real-time in order to display center Cₘ of mass of basket distal assembly 15 and/or the updated locations of electrodes 26 on electro-anatomical map 20 (Fig. 1). Further, the output of electrode location determiner 500 may be utilized for real-time tracking of electrodes 26.

It will be appreciated that, although most of the embodiments have been described herein with reference to a basket type catheter, the system and methods described herein may also be applied to balloon type catheters.

It will also be appreciated that, although most of the embodiments have been described in reference to an ablation catheter including a force sensing device at a distal end of the shaft, the system and methods described herein may also be applied to catheters that otherwise bend without a force sensing device and/or may be applied to diagnostic based catheters with or without force sensing devices on their shaft.

Unless specifically stated otherwise, as apparent from the preceding discussions, it is appreciated that, throughout the specification, discussions utilizing terms such as "processing," "computing," "calculating," "determining," or the like, refer to the action and/or processes of a general purpose computer of any type, such as a client/server system, mobile computing devices, smart appliances, cloud computing units or similar electronic computing devices that manipulate and/or transform data within the computing system's registers and/or memories into other data within the computing system's memories, registers or other such information storage, transmission or display devices.

Embodiments of the present disclosure may include apparatus for performing the operations herein. This apparatus may be specially constructed for the desired purposes, or it may comprise a computing device or system typically having at least one processor and at least one memory, selectively activated or reconfigured by a computer program stored in the computer. The resultant apparatus when instructed by software may turn the general-purpose computer into elements as discussed herein. The instructions may define the device in operation with the computer platform for which it is desired. Such a computer program may be stored in a computer readable storage medium, such as, but not limited to, any type of disk, including optical disks, magnetic-optical disks, read-only memories (ROMs), volatile and non-volatile memories, random access memories (RAMs), electrically programmable read-only memories (EPROMs), electrically erasable and programmable read only memories (EEPROMs), magnetic or optical cards, Flash memory, disk-on-key or any other type of media suitable for storing electronic instructions and capable of being coupled to a computer system bus. The computer readable storage medium may also be implemented in cloud storage.

Some general-purpose computers may comprise at least one communication element to enable communication with a data network and/or a mobile communications network.

The processes and displays presented herein are not inherently related to any particular computer or other apparatus. Various general-purpose systems may be used with programs in accordance with the teachings herein, or it may prove convenient to construct a more specialized apparatus to perform the desired method. In addition, embodiments of the present disclosure are not described with reference to any particular programming language. It will be appreciated that a variety of programming languages may be used to implement the teachings of the invention as described herein.

## Claims

1. A method implemented by a processor (55) of an electro-anatomical mapping system (10) utilizing a catheter (14) having a basket distal assembly comprising a plurality of splines and having multiple electrodes (26) distributed over the splines, the method comprising:
receiving active current locations, ACL, of said electrodes (26);
calculating (520) a center of mass of said distal assembly from the ACL locations of said electrodes (26) by generating an average value of said ACL locations;
using a sensor (29) having a sensor longitudinal axis (530) aligned along a shaft longitudinal axis (86) of a shaft (84) to which said distal assembly is attached and configured to generate a position of the shaft in six degrees of freedom, checking whether or not said shaft longitudinal axis extends through said center of mass;
if not, calculating (526) a tilt angle of said center of mass from said sensor longitudinal axis and a line (540) extending from a position of said sensor and said center of mass; and
correcting said ACL locations according to said tilt angle.

2. The method according to claim 1 wherein said sensor is a magnetic sensor.

3. The method according to any preceding claim wherein said calculating a tilt angle comprises determining an angle between said longitudinal axis and said line.

4. The method according to any preceding claim and also comprising displaying at least one of: corrected said ACL locations and said center of mass on a map of said electro-anatomical mapping system, optionally wherein said displaying is in real-time.

5. The method according to claim 4 and also comprising real-time tracking of at least one of: corrected said ACL locations and said center of mass.

6. The method according to any one of claims 1 to 5, wherein said distal assembly is connected to said shaft via a flexible section in said shaft.

7. The method according to claim 6 wherein said flexible section is one of a helical spring (406) and a joint.

8. An electrode (500) location determiner for an electro-anatomical mapping system utilizing a catheter having a basket distal assembly comprising a plurality of splines and having multiple electrodes (26) distributed over the splines, the determiner comprising:
a center of mass calculator (510) configured to receive active current locations, ACL, of said electrodes (26) and to calculate a center of mass of said distal assembly from said ACL locations of said electrodes by generating an average value of said ACL locations;
a tilt angle calculator (512) configured (i) to receive output from a sensor having a sensor longitudinal axis aligned along a shaft longitudinal axis of a shaft to which said distal assembly is attached and configured to generate a position of the shaft in six degrees of freedom, (ii) to check whether or not said shaft longitudinal axis extends through said center of mass and, if not, (iii) to calculate a tilt angle of said center of mass from said sensor longitudinal axis and a line extending from a position of said sensor and said center of mass; and
an electrode position updater (514) configured to correct said ACL locations according to said tilt angle.

9. The determiner according to claim 8 wherein said sensor is a magnetic sensor.

10. The determiner according to either of claims 8 and 9 said tilt angle calculator to determine an angle between said longitudinal axis and said line.

11. The determiner according to any one of claims 8 to 10 and also comprising a display (27) to display at least one of: corrected said ACL locations and said center of mass on a map of said electro-anatomical mapping system, optionally wherein said display operates in real-time.

12. The determiner according to claim 11 and also comprising a real-time tracker (55) to track at least one of: corrected said ACL locations and said center of mass.

13. The determiner according to any one of claims 8 to 12 wherein said distal assembly is connected to said shaft via a flexible section in said shaft.

14. The determiner according to claim 13, wherein said flexible section is one of a helical spring (406) and a joint.

## Patentansprüche

1. Verfahren, das durch einen Prozessor (55) eines elektroanatomischen Kartierungssystems (10) implementiert wird, das einen Katheter (14) verwendet, der eine distale Korbanordnung aufweist, umfassend eine Vielzahl von Splines und aufweisend mehrere Elektroden (26), die über die Splines verteilt sind, das Verfahren umfassend:
Empfangen aktiver aktueller Standorte, ACL, der Elektroden (26);
Berechnen (520) eines Schwerpunkts der distalen Anordnung aus den ACL-Standorten der Elektroden (26) durch ein Erzeugen eines Durchschnittswerts der ACL-Standorte;
Verwenden eines Sensors (29), der eine Sensorlängsachse (530) aufweist, die entlang einer Schaftlängsachse (86) eines Schafts (84) ausgerichtet ist, an dem die distale Anordnung befestigt ist, und der konfiguriert ist, um eine Position des Schafts in sechs Freiheitsgraden zu erzeugen, wobei überprüft wird, ob sich die Schaftlängsachse durch den Schwerpunkt erstreckt oder nicht;
falls nicht, Berechnen (526) eines Neigungswinkels des Schwerpunkts von der Sensorlängsachse und einer Linie (540), die sich von einer Position des Sensors und des Schwerpunkts erstreckt; und
Korrigieren der ACL-Standorte gemäß dem Neigungswinkel.

2. Verfahren nach Anspruch 1, wobei der Sensor ein magnetischer Sensor ist.

3. Verfahren nach einem der vorstehenden Ansprüche, wobei das Berechnen eines Neigungswinkels ein Bestimmen eines Winkels zwischen der Längsachse und der Linie umfasst.

4. Verfahren nach einem der vorstehenden Ansprüche und ebenso umfassend ein Anzeigen von mindestens einem von: korrigierten der ACL-Standorte und dem Schwerpunkt auf einer Karte des elektroanatomischen Kartierungssystems, optional wobei die Anzeige in Echtzeit erfolgt.

5. Verfahren nach Anspruch 4 und ebenso umfassend die Echtzeitverfolgung von mindestens einem von: korrigierten der ACL-Standorte und dem Schwerpunkt.

6. Verfahren nach einem der Ansprüche 1 bis 5, wobei die distale Anordnung über einen flexiblen Abschnitt in dem Schaft mit dem Schaft verbunden ist.

7. Verfahren nach Anspruch 6, wobei der flexible Abschnitt eines von einer Schraubenfeder (406) und einem Gelenk ist.

8. Standortbestimmer für eine Elektrode (500) für ein elektroanatomisches Kartierungssystem, das einen Katheter verwendet, der eine distale Korbanordnung aufweist, umfassend eine Vielzahl von Splines und aufweisend mehrere Elektroden (26), die über die Splines verteilt sind, der Bestimmer umfassend:
einen Schwerpunktrechner (510), der konfiguriert ist, um aktive aktuelle Standorte, ACL, der Elektroden (26) zu empfangen und einen Schwerpunkt der distalen Anordnung von den ACL-Standorten der Elektroden durch das Erzeugen eines Durchschnittswerts der ACL-Standorte zu berechnen;
einen Neigungswinkelrechner (512), der konfiguriert ist, (i) um eine Ausgabe von einem Sensor zu empfangen, der eine Sensorlängsachse aufweist, die entlang einer Schaftlängsachse eines Schafts ausgerichtet ist, an dem die distale Anordnung befestigt ist, und konfiguriert, um eine Position des Schafts in sechs Freiheitsgraden zu erzeugen, (ii) zu prüfen, ob sich die Schaftlängsachse durch den Schwerpunkt erstreckt oder nicht, und, falls nicht, (iii) einen Neigungswinkel des Schwerpunkts von der Sensorlängsachse und einer Linie zu berechnen, die sich von einer Position des Sensors und des Schwerpunkts erstreckt; und
einen Elektrodenpositionsaktualisierer (514), der konfiguriert ist, um die ACL-Standorte gemäß dem Neigungswinkel zu korrigieren.

9. Bestimmer nach Anspruch 8, wobei der Sensor ein magnetischer Sensor ist.

10. Bestimmer nach einem der Ansprüche 8 oder 9, wobei der Neigungswinkelrechner einen Winkel zwischen der Längsachse und der Linie bestimmt.

11. Bestimmer nach einem der Ansprüche 8 bis 10 und ebenso umfassend eine Anzeige (27), um mindestens eines anzuzeigen von: korrigierten der ACL-Standorte und dem Schwerpunkt auf einer Karte des elektroanatomischen Kartierungssystems, optional wobei die Anzeige in Echtzeit erfolgt.

12. Bestimmer nach Anspruch 11, und ebenso umfassend einen Echtzeit-Verfolger (55), um mindestens eines zu verfolgen von: korrigierten der ACL-Standorte und dem Schwerpunkt.

13. Bestimmer nach einem der Ansprüche 8 bis 12, wobei die distale Anordnung über einen flexiblen Abschnitt in dem Schaft mit dem Schaft verbunden ist.

14. Bestimmer nach Anspruch 13, wobei der flexible Abschnitt eines von einer Schraubenfeder (406) und einem Gelenk ist.

## Revendications

1. Procédé mis en œuvre par un processeur (55) d'un système de cartographie électro-anatomique (10) utilisant un cathéter (14) ayant un ensemble distal de panier comprenant une pluralité de cannelures et ayant de multiples électrodes (26) réparties sur les cannelures, le procédé comprenant :
la réception d'emplacements actuels actifs, ACL, desdites électrodes (26) ;
le calcul (520) d'un centre de masse dudit ensemble distal à partir des emplacements ACL desdites électrodes (26) en générant une valeur moyenne desdits emplacements ACL ;
à l'aide d'un capteur (29) ayant un axe longitudinal de capteur (530) aligné le long d'un axe longitudinal d'arbre (86) d'un arbre (84) auquel ledit ensemble distal est fixé et qui est conçu pour générer une position de l'arbre à six degrés de liberté, le fait de vérifier si ledit axe longitudinal d'arbre s'étend ou non à travers ledit centre de masse ;
dans la négative, le calcul (526) d'un angle d'inclinaison dudit centre de masse par rapport audit axe longitudinal de capteur et à une ligne (540) s'étendant à partir d'une position dudit capteur et dudit centre de masse ; et
la correction desdits emplacements ACL en fonction dudit angle d'inclinaison.

2. Procédé selon la revendication 1, dans lequel ledit capteur est un capteur magnétique.

3. Procédé selon l'une quelconque revendication précédente, dans lequel ledit calcul d'un angle d'inclinaison comprend la détermination d'un angle entre ledit axe longitudinal et ladite ligne.

4. Procédé selon l'une quelconque revendication précédente et comprenant également l'affichage d'au moins l'un parmi : lesdits emplacements ACL corrigés et ledit centre de masse sur une carte dudit système de cartographie électro-anatomique, éventuellement dans lequel ledit affichage se fait en temps réel.

5. Procédé selon la revendication 4 et comprenant également le suivi en temps réel d'au moins l'un parmi : lesdits emplacements ACL corrigés et ledit centre de masse.

6. Procédé selon l'une quelconque des revendications 1 à 5, dans lequel ledit ensemble distal est relié audit arbre par une section flexible dans ledit arbre.

7. Procédé selon la revendication 6, dans lequel ladite section flexible est l'un parmi un ressort hélicoïdal (406) et une articulation.

8. Dispositif de détermination de l'emplacement d'une électrode (500) pour un système de cartographie électro-anatomique utilisant un cathéter ayant un ensemble distal de panier comprenant une pluralité de cannelures et ayant de multiples électrodes (26) réparties sur les cannelures, le dispositif de détermination comprenant :
un calculateur de centre de masse (510) configuré pour recevoir des emplacements actuels actifs, ACL, desdites électrodes (26) et pour calculer un centre de masse dudit ensemble distal
à partir desdits emplacements ACL desdites électrodes en générant une valeur moyenne desdits emplacements ACL ;
un calculateur d'angle d'inclinaison (512) configuré (i) pour recevoir des données à partir d'un capteur ayant un axe longitudinal de capteur aligné le long d'axe longitudinal d'arbre d'un arbre auquel ledit ensemble distal est fixé et conçu pour générer une position de l'arbre à six degrés de liberté, (ii) pour vérifier si ledit axe longitudinal d'arbre s'étend ou non à travers ledit centre de masse et, dans la négative, (iii) pour calculer un angle d'inclinaison dudit centre de masse par rapport audit axe longitudinal de capteur et à une ligne s'étendant de la position dudit capteur et dudit centre de masse ; et
un dispositif de mise à jour de la position des électrodes (514) configuré pour corriger lesdits emplacements ACL en fonction dudit angle d'inclinaison.

9. Appareil selon la revendication 8, dans lequel ledit capteur est un capteur magnétique.

10. Dispositif de détermination selon l'une quelconque des revendications 8 et 9, ledit calculateur d'angle d'inclinaison permettant de déterminer un angle entre ledit axe longitudinal et ladite ligne.

11. Dispositif de détermination selon l'une quelconque des revendications 8 à 10 et comprenant également un dispositif d'affichage (27) pour afficher au moins l'un parmi : lesdits emplacements ACL corrigés et ledit centre de masse sur une carte dudit système de cartographie électro-anatomique, éventuellement dans lequel ledit dispositif d'affichage fonctionne en temps réel.

12. Dispositif de détermination selon la revendication 11 et comprenant également un dispositif de suivi en temps réel (55) pour suivre au moins l'un parmi : lesdits emplacements ACL corrigés et ledit centre de masse.

13. Dispositif de détermination selon l'une quelconque des revendications 8 à 12, dans lequel ledit ensemble distal est relié audit arbre par l'intermédiaire d'une section flexible dans ledit arbre.

14. Dispositif de détermination selon la revendication 13, dans lequel ladite section flexible est l'un parmi un ressort hélicoïdal (406) et une articulation.
